# EUROPEAN PATENT SPECIFICATION

(11) **EP 4 213 764 B1**
(45) Date of publication and mention of the grant of the patent: **10.12.2025**
(21) Application number: 20781738.8
(22) Date of filing: 21.09.2020
(51) Int. Cl.: A61F 2/16

(54) **INTRAOCULAR LENS**
INTRAOKULARLINSE
LENTILLE INTRAOCULAIRE

(43) Date of publication of application: 26.07.2023
(73) Proprietor: Carl Zeiss Meditec AG, 07745 Jena (DE)
(72) Inventor: SCHREIBER, Benjamin, 73447 Oberkochen (DE); BADUR, Thorben, 73447 Oberkochen (DE)
(74) Representative: PATERIS Patentanwälte PartmbB
(86) International application number: PCT/US2020/051800
(87) International publication number: WO 2022/060372

(56) References cited:
- WO-A2-2004/046768
- US-A1- 2019 269 499

## Description

The invention relates to an intraocular lens.

In cataract treatment of an eye, an incision is conventionally made in the cornea of the eye, said incision being large enough to allow a cannula to be inserted through the incision into the eye. After the incision has been made in the cornea, the lens of the eye is broken up by phacoemulsification and then sucked out of the capsular bag of the eye. Thereafter, an intraocular lens is inserted into the capsular bag by means of an injector. The intraocular lens includes an optical body and a haptic element, wherein the haptic element fixes the optical body in the capsular bag.

The haptic element has the function of keeping the optical body as close as possible to the middle of the eye in order to generate an image of maximum quality on the retina of the eye. Moreover, the optical body should be fixed with maximum positional stability in the capsular bag. In addition, the haptic element has the function of stopping the optical body from rotating about its optical axis. This is particularly relevant when the optical body is a toric optical body by means of which cornea curvature is to be corrected, because the toric optical body, if it is arranged in the capsular bag with an incorrect orientation, leads to an imaging aberration on the retina.

Once the intraocular lens has been inserted into the capsular bag, the intraocular lens may be disposed in an incorrect position in the capsular bag. This may be caused, for example, by nonuniform fibrosis. If the intraocular lens has been disposed in the wrong position in the capsular bag, it is necessary to correct the position of the intraocular lens in a surgical intervention or even to change the intraocular lens. Remedy could be provided by an intraocular lens having a correctable position after the insertion of the intraocular lens.

US 2019/0269499 A1 discloses an accommodating intraocular lens. WO 2004/046768 A2 discloses a lens that has optical parameters that may be adjusted in-situ.

The problem addressed by the invention is therefore that of providing an intraocular lens having a correctable position after it has been inserted into a capsular bag of an eye.

The intraocular lens according to the invention comprises an optical body, the latter having an optical axis, a separation plane, in which the optical axis is located and which notionally divides the intraocular lens into a first half and a second half, a first haptic element, the majority of which is disposed in the first half and which has a first conduit, a second haptic element, the majority of which is disposed in the second half and which has a second conduit, a first reservoir, in which a first fluid is disposed and which is disposed in the region of the optical body or in a region adjoining the optical body and the majority of which is disposed in the second half and which is connected to the first conduit, and a second reservoir, in which a second fluid is disposed and which is disposed in the region of the optical body or in a region adjoining the optical body and the majority of which is disposed in the first half and which is connected to the second conduit.

Once the intraocular lens has been inserted into the capsular bag of an eye, the first haptic element and the second haptic element contact the capsular bag. By virtue of reducing the density of the first fluid it is possible to increase the pressure in the first reservoir and in the first conduit. The first haptic element becomes stiffer as a result of an increase in the pressure in the first conduit and, as a result, the optical body moves away from the region of the capsular bag contacting the first haptic element. Analogously, a reduction in the density of the second fluid can bring the optical body to move away from the region of the capsular bag contacting the second haptic element. Reducing the density of the first fluid and/or of the second fluid allows the position of the optical body in the capsular bag to be corrected. Reducing the density of the first fluid and/or of the second fluid are caused by an increase in temperature and/or by a phase transition of the first fluid and/or the second fluid. The phase is understood to mean a spatial region in which the material properties are homogeneous. The phase transition is understood to mean, for example, a transition from liquid to gaseous or a chemical conversion, such as from a dimer to a monomer, for example. The density can be reduced by impinging the first fluid and/or the second fluid with electromagnetic radiation. By way of example, this can be carried out by a physician and by means of a laser. However, it is likewise conceivable for the reduction in density to be implemented by impingement by daylight. Should the optical body not be disposed centrally in the capsular bag, the daylight, which strikes the intraocular lens via the pupil of the eye, is not incident on the intraocular lens in symmetric fashion. As a result, one of the first reservoir and the second reservoir is impinged more strongly with daylight than the other of the first reservoir and the second reservoir, as a result of which the density of the fluid disposed in the one reservoir is reduced to a greater extent than the density of the fluid disposed in the other reservoir. As a result, the optical body is centered independently in the capsular bag.

The first reservoir and the second reservoir are only disposed in a region of the intraocular lens which is situated completely outside of a distance of 0.8*R from the optical axis of the optical body, where R is the distance between the optical axis and the peripheral edge of the optical body. Particularly preferably, the region is situated completely outside of a distance of 0.9*R, in particular a distance of 0.95*R, from the optical axis. As a result, the first reservoir and the second reservoir only impair the visual field slightly.

Preferably, the first haptic element is disposed completely within the first half and/or wherein the second haptic element is disposed completely within the second half. Moreover, preferably, the first reservoir is disposed completely within the second half and/or wherein the second reservoir is disposed completely within the first half.

The intraocular lens preferably has a first connecting conduit, which connects the first reservoir to the first conduit, and the first fluid experiences a cross-sectional constriction in its flow when it flows from the first reservoir into the first connecting conduit and/or the intraocular lens preferably has a second connecting conduit, which connects the second reservoir to the second conduit, and the second fluid experiences a cross-sectional constriction in its flow when it flows from the second reservoir into the second connecting conduit.

Preferably, the first reservoir is permanently connected to the complete first conduit in fluid-conducting fashion and/or wherein the second reservoir is permanently connected to the complete second conduit in fluid-conducting fashion. As a result, the optical body can be permanently centered in the capsular bag in independent fashion.

The intraocular lens preferably has a first shutoff device which has a closed state, in which a flow of the first fluid in a flow direction from the first reservoir into the part of the first conduit, which is disposed downstream in the flow direction, and in reverse is prevented or in which a flow of the first fluid in a flow direction from the first reservoir into the part of the first conduit, which is disposed downstream in the flow direction, is facilitated and prevented in reverse, and which has an open state, in which the flow of the first fluid in the flow direction into the part of the first conduit, which is disposed downstream in the flow direction, and in reverse is facilitated, and/or the intraocular lens preferably has a second shutoff device which has a closed state, in which a flow of the second fluid in a flow direction from the second reservoir into the part of the second conduit, which is disposed downstream in the flow direction, and in reverse is prevented or in which a flow of the second fluid in a flow direction from the second reservoir into the part of the second conduit, which is disposed downstream in the flow direction, is facilitated and prevented in reverse, and which has an open state, in which the flow of the second fluid in the flow direction into the part of the second conduit, which is disposed downstream in the flow direction, and in reverse is facilitated. By means of the first shutoff device and/or the second shutoff device, it is possible to bring the first shutoff device and/or the second shutoff device into the open state at a desired time and subsequently impinge the first fluid and/or the second fluid with electromagnetic radiation in order to center the optical body in the capsular bag. Moreover, by bringing the first shutoff device and/or the second shutoff device into the closed state, it is possible to prevent a return flow of the fluid into the reservoir after the optical body was centered in the capsular bag.

The first shutoff device is preferably disposed in the first haptic element and/or the second shutoff device is preferably disposed in the second haptic element. As a result, the first shutoff device and/or the second shutoff device cannot impair the imaging properties of the optical body.

Preferably, the first shutoff device has a bimetal, which is configured to bring the first shutoff device from the closed state into the open state by way of a temperature increase of the bimetal, and/or the second shutoff device has a bimetal, which is configured to bring the second shutoff device from the closed state into the open state by way of a temperature increase of the bimetal. The temperature of the bimetal can be increased by irradiating the bimetal with electromagnetic radiation, for example by means of a laser. As a result, it is not necessary to introduce a surgical instrument into the capsular bag in order to bring the first shutoff device and/or the second shutoff device into the open state. As a result of the bimetal cooling by thermal conduction, the first shutoff device and/or the second shutoff device automatically returns into the closed state.

The first shutoff device preferably has a photosystem containing an amount of a monomer and/or an amount of a dimer, wherein two of the monomers are set up to enter into a photoaddition with one another and form the dimer as a result and consequently bring the first shutoff device into its open state and the dimer is set up to enter into a photodissociation and form two of the monomers as a result and consequently bring the first shutoff device into its closed state. The second shutoff device preferably has a photosystem containing an amount of a monomer and/or an amount of a dimer, wherein two of the monomers are set up to enter into a photoaddition with one another and form the dimer as a result and consequently bring the second shutoff device into its open state and the dimer is set up to enter into a photodissociation and form two of the monomers as a result and consequently bring the second shutoff device into its closed state. The photosystem of the first shutoff device can have the same compounds as the photosystem of the second shutoff device.

Preferably, the first fluid and/or the second fluid has a photosystem containing an amount of a monomer and/or an amount of a dimer, wherein two of the monomers are set up to enter into a photoaddition with one another and form the dimer as a result and the dimer is set up to enter into a photodissociation and form two of the monomers as a result. The photosystem of the first fluid and the photosystem of the second fluid can have the same compounds. Moreover, the photosystem of the first fluid and/or the photosystem of the second fluid can have the same compounds as the photosystem of the first shutoff device and/or of the second shutoff device.

By way of example, the photoaddition can be a photocycloaddition, in particular a [2+2] photocycloaddition. The photoaddition reactions and, in particular, the photocycloaddition reactions are known to a person skilled in the art (see, for example, Jonathan Clayden, Nick Geeves, Stuart Warren, Organic Chemistry, Oxford University Press, 2006). By way of example, the photosystem may include coumarin, a coumarin derivative, a cinnamic ester, in particular methyl cinnamate, a cinnamic ester derivative, or a stilbene derivative, and/or the dimer of the aforementioned compounds. One example of photoaddition and photodissociation is depicted in reaction equation (1) below for coumarin and the coumarin derivative, with the dimer depicted on the right of the reaction arrow and the monomer on the left of the reaction arrow:

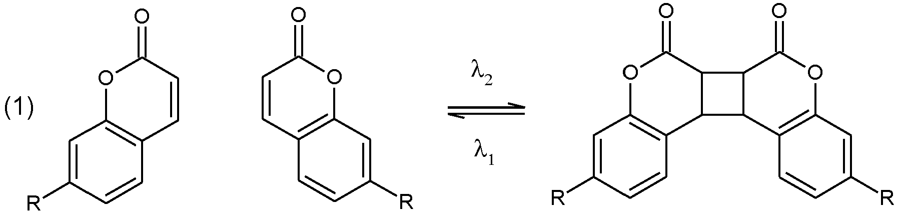

R may be, for example, -H, -NR'₂, -NH₂, -NHCOR', -OH or -OR', where the substance is coumarin when R=-H. R' may be an aliphatic or aromatic radical or an acrylate. Irradiation with a second wavelength λ₂ leads to formation of the dimer; irradiation with a first wavelength λ₁ leads to dissociation of the dimer to give the monomer. The dimer has a higher density than the monomer. Irradiation with the second wavelength λ₂ thus leads to reduction of the pressure of the fluid; irradiation with the first wavelength λ₁ leads to an increase in the pressure of the fluid.

A further example of photoaddition is depicted in reaction equation (2) below for methyl cinnamate, with the dimer depicted on the right of the reaction arrow and the monomer on the left of the reaction arrow:

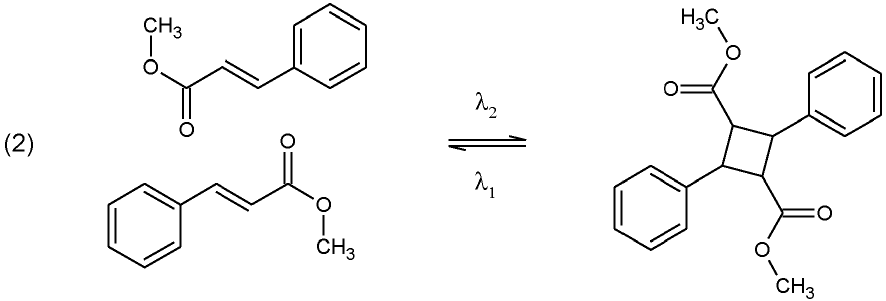

Irradiation with a second wavelength λ₂ leads to formation of the dimer; irradiation with a first wavelength λ₁ leads to dissociation of the dimer to give the monomer. The dimer has a higher density than the monomer. Irradiation with the second wavelength λ₂ thus leads to reduction of the pressure of the fluid; irradiation with the first wavelength λ₁ leads to an increase in the pressure.

A further example of photoaddition and photodissociation is depicted in reaction equation (3) below for a stilbene derivative, with the dimer depicted on the right of the reaction arrow and the monomer on the left of the reaction arrow:

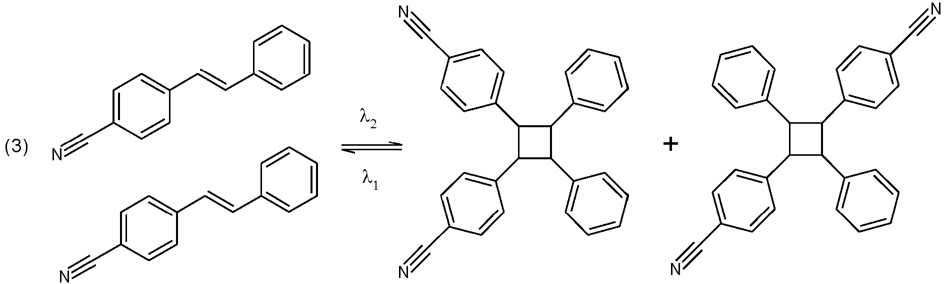

The dimer is present in two isomers. Irradiation with a second wavelength λ₂ leads to formation of the dimer; irradiation with a first wavelength λ₁ leads to dissociation of the dimer to give the monomer. The dimer has a higher density than the monomer. Irradiation with the second wavelength λ₂ thus leads to lowering of the pressure in the conduit; irradiation with the first wavelength λ₁ leads to an increase in the pressure in the conduit.

The intraocular lens preferably has a bandpass filter that is disposed around the photosystem and configured to allow a first wavelength range and a second wavelength range to pass through to the photosystem, wherein an irradiation of the dimer in the first wavelength range leads to dissociation of the dimer and an irradiation of the monomer in the second wavelength range leads to formation of the dimer. In this way, it is possible to prevent an unwanted phase transition of the photosystem resulting from incidence of daylight into the eye. The photosystem may, for example, be mixed with the bandpass filter and/or the bandpass filter may be disposed around the photosystem. Particularly preferably, the first wavelength range includes the first wavelength λ₁ and the second wavelength range includes the second wavelength λ₂.

Preferably, the intraocular lens has a filter that is disposed around the photosystem and configured to allow a first wavelength range to pass through to the photosystem and to block a second wavelength range, wherein an irradiation of the dimer in the first wavelength range leads to dissociation of the dimer and an irradiation of the monomer in the second wavelength range leads to formation of the dimer.

The first haptic element is preferably C-shaped and/or the second haptic element is preferably C-shaped.

The invention is elucidated in detail below with reference to the schematic drawings appended.
Figure 1 shows a plan view of a preferred embodiment of the intraocular lens according to the invention.
Figure 2 illustrates how the intraocular lens can be centered, in exemplary fashion using the preferred embodiment.
Figure 3 shows a first embodiment of a shutoff device of the intraocular lens.
Figure 4 shows a second embodiment of a shutoff device of the intraocular lens.
Figure 5 shows a third embodiment of a shutoff device of the intraocular lens.
Figure 6 shows a fourth embodiment of a shutoff device of the intraocular lens.
Figure 7 shows an exemplary bandpass filter for the intraocular lens.

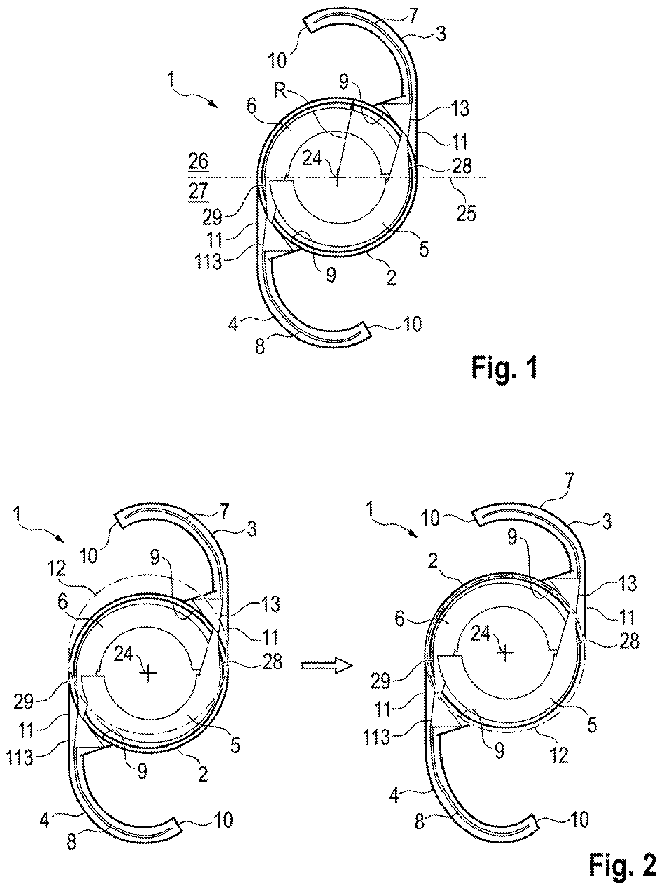

As is evident from figures 1 and 2, an intraocular lens 1 according to the invention comprises an optical body 2, a first haptic element 3, a second haptic element 4, a first reservoir 5, a second reservoir 6 and a separation plane 25. The optical body 2 has an optical axis 24, which is located in the separation plane 25. The separation plane 25 notionally divides the intraocular lens 1 into a first half 26 and a second half 27. The majority of the first haptic element 3 is disposed in the first half 26 and said first haptic element has a first conduit 7. The majority of the second haptic element 4 is disposed in the second half 27 and said second haptic element has a second conduit 8. A first fluid is disposed in the first reservoir 5 and the first reservoir 5 is disposed in the region of the optical body 2 or in a region adjoining the optical body 2 and the majority of said reservoir is disposed in the second half 27 and said reservoir is connected to the first conduit 7. A second fluid is disposed in the second reservoir 6 and the second reservoir 6 is disposed in the region of the optical body 2 or in a region adjoining the optical body 2 and the majority of said reservoir is disposed in the first half 26 and said reservoir is connected to the second conduit 8. The phrase "the majority of" should be understood to mean that a greater volume of the first haptic element 3 is located in the first half 26 than in the second half 27 and that a greater volume of the second haptic element 4 is located in the second half 27 than in the first half 26.

The first reservoir 5 and the second reservoir 6 could be only disposed in a region of the intraocular lens 1 which is situated completely outside of a distance of 0.8*R from the optical axis 24 of the optical body 2, where R is the distance between the optical axis 24 and the peripheral edge of the optical body 2, as also plotted in figure 1. The region can also be situated completely outside of a distance of 0.9*R, in particular a distance of 0.95*R, from the optical axis 24.

Figures 1 and 2 show that the first reservoir 5 can be formed in point-symmetric fashion with respect to the second reservoir 6, wherein the point of symmetry may be located on the optical axis 24. Moreover, it is possible to identify that the first haptic element 3 can be shaped in point-symmetric and/or axisymmetric fashion with respect to the second haptic element 4, wherein the point of symmetry is located on the optical axis 24 and/or the axis of symmetry coincides with the optical axis 24. Moreover, it is conceivable for the first haptic element 3 to be C-shaped and/or for the second haptic element 4 to be C-shaped. The first haptic element 3 and/or the second haptic element 4 can each have a haptic longitudinal end 9 disposed at the optical body 2 and a haptic longitudinal end 10 distant from the optical body 2.

As is evident from figures 1 and 2, the first haptic element 3 can be disposed completely within the first half 26 and/or the second haptic element 3 can be disposed completely within the second half 26. Moreover, it is evident that the first reservoir 5 can be disposed completely within the second half 27 and/or that the second reservoir 6 can be disposed completely within the first half 26.

Moreover, figures 1 and 2 show that the intraocular lens 1 can have a first connecting conduit 28, which connects the first reservoir 5 to the first conduit 7, and the first fluid experiences a cross-sectional constriction in its flow when it flows from the first reservoir 5 into the first connecting conduit 28 and/or that the intraocular lens 1 can have a second connecting conduit 29, which connects the second reservoir 6 to the second conduit 8, and the second fluid experiences a cross-sectional constriction in its flow when it flows from the second reservoir 6 into the second connecting conduit 29. Here, it is conceivable for the first connecting conduit 28 to extend in the optical body 2 and in the first half 26 and/or for the second connecting conduit 29 to extend in the optical body 2 and in the second half 27.

By way of example, the first haptic element 3 and/or the second haptic element 4 can have an elastomer or consist of the elastomer. The elastomer can be identical to the material of the optical body 2 and, for example, have a silicone and/or polyacrylate or consist of the silicone and/or the polyacrylate.

It is conceivable for the first reservoir 5 to be permanently connected to the first conduit 7 in fluid-conducting fashion and/or for the second reservoir 6 to be permanently connected to the second conduit 8 in fluid-conducting fashion. This means that the first fluid can flow from the first reservoir 5 into the complete first conduit 7 and in reverse at all times and/or that the second fluid can flow from the second reservoir 6 into the complete second conduit 8 and in reverse at all times.

Alternatively, it is conceivable that the intraocular lens 1 has a first shutoff device 13 which has a closed state, in which a flow of the first fluid in a flow direction from the first reservoir 5 into the part of the first conduit 8, which is disposed downstream in the flow direction, and in reverse is prevented or in which a flow of the first fluid in a flow direction from the first reservoir 5 into the part of the first conduit 8, which is disposed downstream in the flow direction, is facilitated and prevented in reverse, and which has an open state, in which the flow of the first fluid in the flow direction into the part of the first conduit 8, which is disposed downstream in the flow direction, and in reverse is facilitated, and/or that the intraocular lens 1 has a second shutoff device 113 which has a closed state, in which a flow of the second fluid in a flow direction from the second reservoir 6 into the part of the second conduit 9, which is disposed downstream in the flow direction, and in reverse is prevented or in which a flow of the second fluid in a flow direction from the second reservoir 6 into the part of the second conduit 9, which is disposed downstream in the flow direction, is facilitated and prevented in reverse, and which has an open state, in which the flow of the second fluid in the flow direction into the part of the second conduit 9, which is disposed downstream in the flow direction, and in reverse is facilitated.

Figure 2 illustrates how the intraocular lens 1 can center itself. To this end, the assumption is made that the first reservoir 5 is permanently connected to the first conduit 7 in fluid-conducting fashion and that the second reservoir 6 is permanently connected to the second conduit 8 in fluid-conducting fashion or that the first shutoff device 13 and the second shutoff device are in their open states. The left-hand image of figure 2 illustrates that the optical body 2 is not disposed centrally in a capsular bag of an eye. An illumination spot 12 is illustrated on the intraocular lens 1; it arises as a result of light entering into the capsular bag via the pupil of the eye. As a result of the optical body 2 not being disposed centrally in the capsular bag, the optical body 2 is not illuminated uniformly by the illumination spot 12, as a result of which, in the exemplary case of figure 2, the second reservoir 6 captures more of the light of the illumination spot 12 than the first reservoir 5. As a result, the density of the second fluid reduces more strongly than the density of the first fluid, as a result of which the pressure in the second reservoir 6 and in the second conduit 8 increases to a greater extent than in the first reservoir 5 and the first conduit 7. As a result, the second haptic element 4 stiffens more than the first haptic element 3, as a result of which the optical body 2 moves away from the longitudinal end 10 of the second haptic element 4 distant from the optical body 2 and moves toward the longitudinal end 10 of the first haptic element 3 distant from the optical body 2. Hence, the optical body 2 is centered independently in the capsular bag, with the intraocular lens 1 with the optical body 2 centered in the capsular bag being illustrated in the right-hand image of figure 2. In particular, this is identifiable on the basis of the illumination spot 12, which illuminates the optical body 2 in almost symmetrical fashion.

Figures 1 and 2 show that the first shutoff device 13 can be disposed in the first haptic element 3 and/or that the second shutoff device 113 can be disposed in the second haptic element 4. To this end, the first haptic element 3 and/or the second haptic element 4 can each have a haptic thickening 11, in which the associated shutoff device 13, 113 is disposed. The haptic thickening 11 can be disposed at the haptic longitudinal end 9 which is disposed at the optical body 2. Here, it is conceivable for the conduit 7, 8 to be disposed completely downstream of the associated shutoff device 13, 113 in the flow direction out of the reservoir 5, 6, as also illustrated in figures 1 and 2. Alternatively, it is conceivable for a longer section of the conduit 7, 8 to be disposed downstream of the associated shutoff device 13, 113 in the flow direction out of the reservoir 5, 6; in particular, at least 70% of the length of the conduit 7, 8 are disposed downstream of the associated shutoff device 13, 113 in the flow direction.

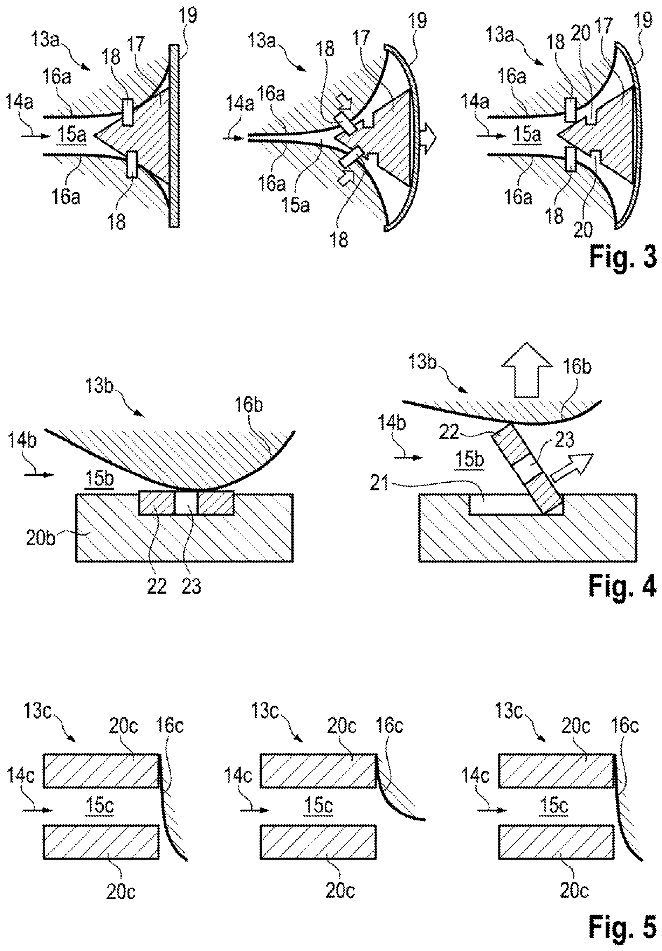

Figures 3 to 6 illustrate four embodiments of the shutoff device 13, 113. In the first to third embodiment as per figures 3 to 5, the first shutoff device 13 has a bimetal 16, which is configured to bring the first shutoff device 13 from the closed state into the open state by way of a temperature increase of the bimetal 16, and/or the second shutoff device 113 has a bimetal 16, which is configured to bring the second shutoff device 113 from the closed state into the open state by way of a temperature increase of the bimetal 16.

According to the first embodiment of the shutoff device 13a as per figure 3, the shutoff device 13a has a shutoff device conduit 15a, into which the fluid enters from the reservoir 5, 6 in the flow direction 14a and the cross section of which opens out in the flow direction 14a. The shutoff device conduit 15a is formed by the bimetal 16a. At the end side of the shutoff device conduit 15a, the shutoff device 13a has a plug 17 which is configured to block the shutoff device conduit 15a in the region where the shutoff device conduit 15 widens so that the fluid cannot emerge from the shutoff device 13a. Figure 3 illustrates the shutoff device 13a at three different times, with time advancing from left to right. At the first time, the shutoff device 13a is blocked by the plug 17 and the shutoff device 13a is in its closed state; the shutoff device 13a is brought into the open state at the second time and the shutoff device 13a is in its open state at the third time. The shutoff device 13a has a bar 18, which is fastened to the bimetal 16a and which engages in a plug cutout 20 in the plug 17 in the closed state. In order to bring the shutoff device 13a into the open state, the bimetal 16a should be heated, as a result of which its curvature increases in the region of the shutoff device conduit 15a where the cross section increases, as a result of which the bar departs from the plug cutout 20, as illustrated for the second time. In the open state, the bar 18 keeps the plug at a distance from the bimetal. The flow 14a presses the plug away from the bar 18 at the third time and consequently there is a flow between the bar 18 and the plug 17. It is conceivable for the shutoff device 13a to function like a valve because a flow counter to 14a leads to the plug 17 being pressed onto the bar 18 and consequently the fluid being unable to pass the shutoff device counter to the flow direction 14a. The shutoff device 13a can have a holding part 19, which is configured to pretension the plug 17 in the direction counter to the flow direction 14a. By way of example, the holding part 19 could have one or more bands, with the fluid being able to pass the holding part 19 next to and/or between the bands.

According to the second embodiment of the shutoff device 13b as per figure 4, the shutoff device 13b has a shutoff device conduit 15b, into which the fluid enters from the reservoir 5, 6 in the flow direction 14b. In figure 4, the shutoff device 13b is illustrated in the closed state in the left-hand image and in the open state in the right-hand image. The shutoff device 13b has a wall 20b delimiting the shutoff device conduit 15b, the wall having a wall cutout 21 in which a lever 22 is disposed in the closed state. At one longitudinal end of the lever 22, the lever 22 is attached to the wall 20b. The other longitudinal end of the lever 22 is pretensioned away from the wall by means of a pretensioning means. The bimetal 16b delimits the shutoff device conduit 15b opposite to the wall cutout 21. In the closed state, the bimetal 16b presses the lever 22 into the wall cutout 21. In the open state, the bimetal 16b has a lower curvature than in the closed state, and so the lever 22 is pressed out of the wall cutout 21 by the pretensioning means. The lever 22 can have a lever through-hole 23, through which the fluid can flow in the open state. According to the second embodiment of the shutoff device 13b, the shutoff device can be switched as desired between the open state and the closed state.

According to the third embodiment of the shutoff device 13c as per figure 5, the shutoff device 13c has a shutoff device conduit 15c, into which the fluid enters from the reservoir 5, 6 in the flow direction 14c. The shutoff device conduit 15c is delimited by a wall 20c in the circumferential direction thereof. The bimetal 16c is disposed at the end side of the shutoff device conduit 15c, said bimetal being configured to open and close the end side by way of its curvature and consequently switch the shutoff device between its open state and its closed state.

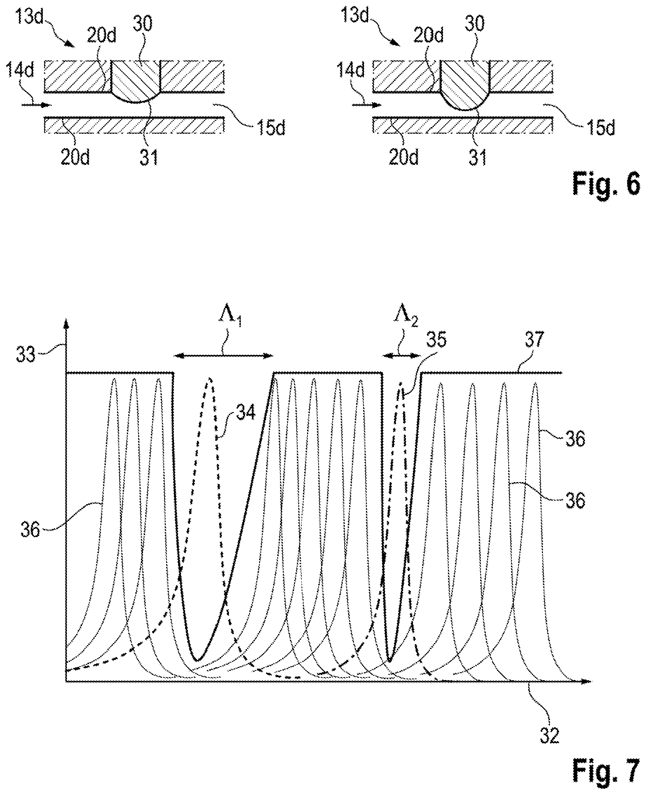

In the fourth embodiment of the shutoff device 13, 113 as per figure 6, the first shutoff device 13 has a photosystem 30 containing an amount of a monomer and/or an amount of a dimer, wherein two of the monomers are set up to enter into a photoaddition with one another and form the dimer as a result and consequently bring the first shutoff device 13 into its open state and the dimer is set up to enter into a photodissociation and form two of the monomers as a result and consequently bring the first shutoff device 13 into its closed state, and/or the second shutoff device 113 has a photosystem 30 containing an amount of a monomer and/or an amount of a dimer, wherein two of the monomers are set up to enter into a photoaddition with one another and form the dimer as a result and consequently bring the second shutoff device 113 into its open state and the dimer is set up to enter into a photodissociation and form two of the monomers as a result and consequently bring the second shutoff device 113 into its closed state. According to the fourth embodiment of the shutoff device 13d as per figure 5, the shutoff device 13d has a shutoff device conduit 15d, into which the fluid enters from the reservoir 5, 6 in the flow direction 14d. The shutoff device 13d has a wall 20d, which delimits the shutoff device conduit 15d. The wall 20d has a cutout, in which the photosystem 30 has been introduced. A flexible wall 31 delimits the shutoff device conduit 15d in the region of the cutout. By reducing the density of the photosystem 30, the flexible wall 31 can be deformed to such an extent that it contacts the section of the wall 20d facing the cutout and consequently closes the shutoff device conduit 15d. As a result, the shutoff device 13d can be brought into its closed state and into its open state.

It is conceivable for the first fluid and/or the second fluid to have a photosystem containing an amount of a monomer and/or an amount of a dimer, wherein two of the monomers are set up to enter into a photoaddition with one another and form the dimer as a result and the dimer is set up to enter into a photodissociation and form two of the monomers as a result. It is moreover conceivable for the photosystem of the first and/or second fluid to have the same compounds as the photosystem of the first shutoff device 13 and of the second shutoff device 113. By way of example, the photoaddition can be a photocycloaddition, in particular a [2+2] photocycloaddition.

It is moreover conceivable for the intraocular lens 1 to have a bandpass filter that is disposed around the photosystem and configured to allow a first wavelength range Λ₁ and a second wavelength range Λ₂ to pass through to the photosystem, wherein an irradiation of the dimer in the first wavelength range Λ₁ leads to dissociation of the dimer and an irradiation of the monomer in the second wavelength range Λ₂ leads to formation of the dimer.

Figure 7 shows an example of the absorption spectrum of the bandpass filter. The wavelength 32 is plotted across the abscissa, and the absorption 33 is plotted across the ordinate. The bandpass filter has a multitude of different absorbers that each have an individual absorption band 36. All the individual absorption bands 36 cumulatively result in an overall absorption 37 of the bandpass filter. The overall absorption 37 is smaller in the first wavelength range Λ₁ and in the second wavelength range Λ₂ than in the adjacent wavelength ranges. The absorption band 34 of the dimer is disposed in the first wavelength range Λ₁, and the absorption band 35 of the monomer is disposed in the second wavelength range Λ₂. Figure 7 shows that the first wavelength range Λ₁ and the second wavelength range Λ₂ are separated from one another. Alternatively, it is conceivable for the first wavelength range Λ₁ and the second wavelength range Λ₂ to overlap one another.

### List of reference signs

1 Intraocular lens
2 Optical body
3 First haptic element
4 Second haptic element
5 First reservoir
6 Second reservoir
7 First conduit
8 Second conduit
9 Haptic longitudinal end disposed at the optical body
10 Haptic longitudinal end distant from the optical body
11 Haptic thickening
12 Illumination spot
13 First shutoff device
113 Second shutoff device
13a Shutoff device
13b Shutoff device
13c Shutoff device
13d Shutoff device
14a Flow direction
14b Flow direction
14c Flow direction
14d Flow direction
15a Shutoff device conduit
15b Shutoff device conduit
15c Shutoff device conduit
15d Shutoff device conduit
16a Bimetal
16b Bimetal
16c Bimetal
17 Plug
18 Bar
19 Holding part
20 Plug cutout
20b Wall
20c Wall
20d Wall
21 Wall cutout
22 Lever
23 Lever through-hole
24 Optical axis
25 Separation plane
26 First half
27 Second half
28 First connecting conduit
29 Second connecting conduit
30 Photosystem
31 Flexible wall
32 Wavelength
33 Absorption
34 Dimer absorption band
35 Monomer absorption band
36 Individual absorption band
37 Overall absorption
λ₁ First wavelength
λ₂ Second wavelength
Λ₁ First wavelength range
Λ₂ Second wavelength range
R Optical axis - peripheral edge distance

## Claims

1. An intraocular lens comprising an optical body (2), the latter having an optical axis (24), comprising a separation plane (25), in which the optical axis (24) is located and which notionally divides the intraocular lens (1) into a first half (26) and a second half (27), comprising a first haptic element (3), the majority of which is disposed in the first half (26) and which has a first conduit (7), comprising a second haptic element (4), the majority of which is disposed in the second half (27) and which has a second conduit (8), **characterized in that** the intraocular lens (1) comprises a first reservoir (5), in which a first fluid is disposed and which is disposed in the region of the optical body (2) or in a region adjoining the optical body (2) and the majority of which is disposed in the second half (27) and which is connected to the first conduit (7), and a second reservoir (6), in which a second fluid is disposed and which is disposed in the region of the optical body (2) or in a region adjoining the optical body (2) and the majority of which is disposed in the first half (26) and which is connected to the second conduit (7), wherein the first fluid is adapted such that the density of the first fluid can be reduced by an increase in temperature and/or by a phase transition of the first fluid and the second fluid is adapted such that the density of the second fluid can be reduced by an increase in temperature and/or by a phase transition of the second fluid.

2. The intraocular lens as claimed in claim 1, wherein the first reservoir (5) and the second reservoir (6) are only disposed in a region of the intraocular lens (1) which is situated completely outside of a distance of 0.8*R from the optical axis (24) of the optical body (2), where R is the distance between the optical axis (24) and the peripheral edge of the optical body (2).

3. The intraocular lens as claimed in claim 1 or 2, wherein the first haptic element (3) is disposed completely within the first half (26) and/or wherein the second haptic element (4) is disposed completely within the second half (27).

4. The intraocular lens as claimed in any one of claims 1 to 3, wherein the first reservoir (5) is disposed completely within the second half (27) and/or wherein the second reservoir (6) is disposed completely within the first half (26).

5. The intraocular lens as claimed in any one of claims 1 to 4, wherein the intraocular lens (1) has a first connecting conduit (28), which connects the first reservoir (5) to the first conduit (7), and the first fluid experiences a cross-sectional constriction in its flow when it flows from the first reservoir (5) into the first connecting conduit (28) and/or wherein the intraocular lens (1) has a second connecting conduit (29), which connects the second reservoir (6) to the second conduit (8), and the second fluid experiences a cross-sectional constriction in its flow when it flows from the second reservoir (6) into the second connecting conduit (29).

6. The intraocular lens as claimed in any one of claims 1 to 5, wherein the first reservoir (5) is permanently connected to the first conduit (7) in fluid-conducting fashion and/or wherein the second reservoir (6) is permanently connected to the second conduit (8) in fluid-conducting fashion.

7. The intraocular lens as claimed in any one of claims 1 to 6, the first fluid and/or the second fluid has a photosystem containing an amount of a monomer and/or an amount of a dimer, wherein two of the monomers are set up to enter into a photoaddition with one another and form the dimer as a result and the dimer is set up to enter into a photodissociation and form two of the monomers as a result, wherein the photosystem includes coumarin, a coumarin derivative, a cinnamic ester, in particular methyl cinnamate, a cinnamic ester derivative, or a stilbene derivative.

8. The intraocular lens as claimed in claim 7, wherein the intraocular lens (1) has a bandpass filter that is disposed around the photosystem and configured to allow a first wavelength range (Λ₁) and a second wavelength range (Λ₂) to pass through to the photosystem, wherein an irradiation of the dimer in the first wavelength range (Λ₁) leads to dissociation of the dimer and an irradiation of the monomer in the second wavelength range (Λ₂) leads to formation of the dimer.

9. The intraocular lens as claimed in claim 7, wherein the intraocular lens (1) has a filter that is disposed around the photosystem and configured to allow a first wavelength range (Λ₁) to pass through to the photosystem and to block a second wavelength range (Λ₂), wherein an irradiation of the dimer in the first wavelength range (Λ₁) leads to dissociation of the dimer and an irradiation of the monomer in the second wavelength range (Λ₂) leads to formation of the dimer.

10. The intraocular lens as claimed in any one of claims 1 to 9, wherein the first haptic element (3) is C-shaped and/or the second haptic element (4) is C-shaped.

## Patentansprüche

1. Intraokularlinse, umfassend einen Optikkörper (2), wobei letzterer eine optische Achse (24) aufweist, umfassend eine Trennebene (25), in der sich die optische Achse (24) befindet und welche die Intraokularlinse (1) gedanklich in eine erste Hälfte (26) und eine zweite Hälfte (27) unterteilt, umfassend ein erstes haptisches Element (3), dessen überwiegender Teil in der ersten Hälfte (26) angeordnet ist und das eine erste Leitung (7) aufweist, umfassend ein zweites haptisches Element (4), dessen überwiegender Teil in der zweiten Hälfte (27) angeordnet ist und das eine zweite Leitung (8) aufweist, **dadurch gekennzeichnet, dass** die Intraokularlinse (1) ein erstes Reservoir (5) umfasst, in dem ein erstes Fluid angeordnet ist und das in der Region des optischen Körpers (2) oder in einer an den optischen Körper (2) angrenzenden Region angeordnet ist, und wobei dessen überwiegender Teil in der zweiten Hälfte (27) angeordnet ist und mit der ersten Leitung (7) verbunden ist, und ein zweites Reservoir (6) umfasst, in dem ein zweites Fluid angeordnet ist und das in der Region des optischen Körpers (2) oder in einer an den optischen Körper (2) angrenzenden Region angeordnet ist und dessen überwiegender Teil in der ersten Hälfte (26) angeordnet ist und das mit der zweiten Leitung (7) verbunden ist, wobei das erste Fluid derart adaptiert ist, dass die Dichte des ersten Fluids durch eine Erhöhung der Temperatur und/oder durch einen Phasenübergang des ersten Fluids reduziert werden kann, und das zweite Fluid derart adaptiert ist, dass die Dichte des zweiten Fluids durch eine Erhöhung der Temperatur und/oder durch einen Phasenübergang des zweiten Fluids reduziert werden kann.

2. Intraokularlinse nach Anspruch 1, wobei das erste Reservoir (5) und das zweite Reservoir (6) nur in einer Region der Intraokularlinse (1) angeordnet sind, die vollständig außerhalb eines Abstands von 0,8*R zu der optischen Achse (24) des optischen Körpers (2) liegt, wobei R der Abstand zwischen der optischen Achse (24) und dem Umfangsrand des optischen Körpers (2) ist.

3. Intraokularlinse nach Anspruch 1 oder 2, wobei das erste haptische Element (3) vollständig innerhalb der ersten Hälfte (26) angeordnet ist und/oder wobei das zweite haptische Element (4) vollständig innerhalb der zweiten Hälfte (27) angeordnet ist.

4. Intraokularlinse nach einem der Ansprüche 1 bis 3, wobei das erste Reservoir (5) vollständig innerhalb der zweiten Hälfte (27) angeordnet ist und/oder wobei das zweite Reservoir (6) vollständig innerhalb der ersten Hälfte (26) angeordnet ist.

5. Intraokularlinse nach einem der Ansprüche 1 bis 4, wobei die Intraokularlinse (1) eine erste Verbindungsleitung (28) aufweist, die das erste Reservoir (5) mit der ersten Leitung (7) verbindet, und das erste Fluid eine Querschnittsverengung in seiner Strömung erfährt, wenn es aus dem ersten Reservoir (5) in die erste Verbindungsleitung (28) strömt, und/oder wobei die Intraokularlinse (1) eine zweite Verbindungsleitung (29) aufweist, die das zweite Reservoir (6) mit der zweiten Leitung (8) verbindet, und das zweite Fluid eine Querschnittsverengung in seiner Strömung erfährt, wenn es aus dem zweiten Reservoir (6) in die zweite Verbindungsleitung (29) strömt.

6. Intraokularlinse nach einem der Ansprüche 1 bis 5, wobei das erste Reservoir (5) fluidleitend permanent mit der ersten Leitung (7) verbunden ist und/oder wobei das zweite Reservoir (6) fluidleitend permanent mit der zweiten Leitung (8) verbunden ist.

7. Intraokularlinse nach einem der Ansprüche 1 bis 6, wobei das erste Fluid und/oder das zweite Fluid ein Photosystem aufweist/aufweisen, das eine Menge eines Monomers und/oder eine Menge eines Dimers enthält, wobei zwei der Monomere so eingerichtet sind, dass sie miteinander eine Photoaddition eingehen und dadurch das Dimer bilden, und das Dimer so eingerichtet ist, dass es eine Photodissoziation eingeht und dadurch zwei der Monomere bildet, wobei das Photosystem Cumarin, ein Cumarinderivat, einen Zimtsäureester, insbesondere Methylcinnamat, ein Zimtsäureesterderivat oder ein Stilbenderivat einschließt.

8. Intraokularlinse nach Anspruch 7, wobei die Intraokularlinse (1) ein Bandpassfilter aufweist, das um das Photosystem angeordnet ist und ausgelegt ist, um zu ermöglichen, dass ein erster Wellenlängenbereich (Λ₁) und ein zweiter Wellenlängenbereich (Λ₂) das Photosystem passieren, wobei eine Bestrahlung des Dimers in dem ersten Wellenlängenbereich (Λ₁) zur Dissoziation des Dimers führt und eine Bestrahlung des Monomers in dem zweiten Wellenlängenbereich (Λ₂) zur Bildung des Dimers führt.

9. Intraokularlinse nach Anspruch 7, wobei die Intraokularlinse (1) ein Filter aufweist, das um das Photosystem angeordnet ist und ausgelegt ist, um zu ermöglichen, dass ein erster Wellenlängenbereich (Λ₁) das Photosystem passiert und ein zweiter Wellenlängenbereich (Λ₂) blockiert wird, wobei eine Bestrahlung des Dimers in dem ersten Wellenlängenbereich (Λ₁) zur Dissoziation des Dimers führt und eine Bestrahlung des Monomers in dem zweiten Wellenlängenbereich (Λ₂) zur Bildung des Dimers führt.

10. Intraokularlinse nach einem der Ansprüche 1 bis 9, wobei das erste haptische Element (3) C-förmig ist und/oder das zweite haptische Element (4) C-förmig ist.

## Revendications

1. Lentille intraoculaire comprenant un corps optique (2), ce dernier ayant un axe optique (24), comprenant un plan de séparation (25) dans lequel se situe l'axe optique (24) et qui divise théoriquement la lentille intraoculaire (1) en une première moitié (26) et une deuxième moitié (27), comprenant un premier élément haptique (3) dont la majorité est disposée dans la première moitié (26) et qui comporte une première conduite (7), comprenant un deuxième élément haptique (4) dont la majorité est disposée dans la deuxième moitié (27) et qui comporte une deuxième conduite (8), **caractérisée en ce que** la lentille intraoculaire (1) comprend un premier réservoir (5) dans lequel est disposé un premier fluide et qui est disposé dans la région du corps optique (2) ou dans une région jouxtant le corps optique (2) et dont la majorité est disposée dans la deuxième moitié (27) et qui est raccordé à la première conduite (7), et un deuxième réservoir (6) dans lequel est disposé un deuxième fluide et qui est disposé dans la région du corps optique (2) ou dans une région jouxtant le corps optique (2) et dont la majorité est disposée dans la première moitié (26) et qui est raccordé à la deuxième conduite (7), dans laquelle le premier fluide est adapté de telle sorte que la masse volumique du premier fluide peut être réduite par une augmentation de température et/ou par une transition de phase du premier fluide et le deuxième fluide est adapté de telle sorte que la masse volumique du deuxième fluide peut être réduite par une augmentation de température et/ou par une transition de phase du deuxième fluide.

2. Lentille intraoculaire selon la revendication 1, dans laquelle le premier réservoir (5) et le deuxième réservoir (6) sont disposés uniquement dans une région de la lentille intraoculaire (1) qui se situe entièrement au-delà d'une distance de 0,8*R de l'axe optique (24) du corps optique (2), R étant la distance entre l'axe optique (24) et le bord périphérique du corps optique (2).

3. Lentille intraoculaire selon la revendication 1 ou 2, dans laquelle le premier élément haptique (3) est entièrement disposé à l'intérieur de la première moitié (26) et/ou dans laquelle le deuxième élément haptique (4) est entièrement disposé à l'intérieur de la deuxième moitié (27).

4. Lentille intraoculaire selon l'une quelconque des revendications 1 à 3, dans laquelle le premier réservoir (5) est entièrement disposé à l'intérieur de la deuxième moitié (27) et/ou dans laquelle le deuxième réservoir (6) est entièrement disposé à l'intérieur de la première moitié (26).

5. Lentille intraoculaire selon l'une quelconque des revendications 1 à 4, la lentille intraoculaire (1) comportant une première conduite de raccordement (28) qui raccorde le premier réservoir (5) à la première conduite (7) et le premier fluide subissant un rétrécissement de section de son écoulement lorsqu'il s'écoule depuis le premier réservoir (5) à l'intérieur de la première conduite de raccordement (28), et/ou la lentille intraoculaire (1) comportant une deuxième conduite de raccordement (29) qui raccorde le deuxième réservoir (6) à la deuxième conduite (8) et le deuxième fluide subissant un rétrécissement de section de son écoulement lorsqu'il s'écoule depuis le deuxième réservoir (6) à l'intérieur de la deuxième conduite de raccordement (29).

6. Lentille intraoculaire selon l'une quelconque des revendications 1 à 5, dans laquelle le premier réservoir (5) est raccordé en permanence à la première conduite (7) avec capacité d'acheminement de fluide et/ou dans laquelle le deuxième réservoir (6) est raccordé en permanence à la deuxième conduite (8) avec capacité d'acheminement de fluide.

7. Lentille intraoculaire selon l'une quelconque des revendications 1 à 6, dans laquelle le premier fluide et/ou le deuxième fluide comportent un photosystème contenant une certaine quantité d'un monomère et/ou une certaine quantité d'un dimère, deux des monomères étant configurés pour entrer en photoaddition l'un avec l'autre et former le dimère en conséquence et le dimère étant configuré pour entrer en photodissociation et former deux des monomères en conséquence, le photosystème comportant de la coumarine, un dérivé de coumarine, un ester cinnamique, en particulier du cinnamate de méthyle, un dérivé d'ester cinnamique ou un dérivé de stilbène.

8. Lentille intraoculaire selon la revendication 7, la lentille intraoculaire (1) comportant un filtre passebande qui est disposé autour du photosystème et conçu pour laisser passer une première plage de longueurs d'onde (Λ₁) et une deuxième plage de longueurs d'onde (Λ₂) à travers le photosystème, une irradiation du dimère dans la première plage de longueurs d'onde (Λ₁) conduisant à la dissociation du dimère et une irradiation du monomère dans la deuxième plage de longueurs d'onde (Λ₂) conduisant à la formation du dimère.

9. Lentille intraoculaire selon la revendication 7, la lentille intraoculaire (1) comportant un filtre qui est disposé autour du photosystème et conçu pour laisser passer une première plage de longueurs d'onde (Λ₁) à travers le photosystème et pour bloquer une deuxième plage de longueurs d'onde (Λ₂), une irradiation du dimère dans la première plage de longueurs d'onde (Λ₁) conduisant à la dissociation du dimère et une irradiation du monomère dans la deuxième plage de longueurs d'onde (Λ₂) conduisant à la formation du dimère.

10. Lentille intraoculaire selon l'une quelconque des revendications 1 à 9, dans laquelle le premier élément haptique (3) est en forme de C et/ou le deuxième élément haptique (4) est en forme de C.
